# EUROPÄISCHE PATENTSCHRIFT

(11) **EP 0 930 360 B1**
(45) Veröffentlichungstag und Bekanntmachung des Hinweises auf die Patenterteilung: **14.02.2007**
(21) Anmeldenummer: 98811181.1
(22) Anmeldetag: 27.11.1998
(51) Int. Cl.: C12M 1/26

(54) **Modul für mikrobiologische Beurteilungen**
Microbiological diagnostical device
Dispositif de diagnostique microbiologique

(30) Priorität: 01.12.1997 CH 277097
(43) Veröffentlichungstag der Anmeldung: 21.07.1999
(73) Patentinhaber: Empa, 9015 St. Gallen (CH)
(72) Erfinder: Fäh, Daniel, 9053 Teufen AR (CH)
(74) Vertreter: Kulhavy, Sava

(56) Entgegenhaltungen:
- EP-A- 0 013 748
- US-A- 3 203 870
- PITZURRA ET AL: 'Eine neue Methode zur Untersuchung der mikrobiellen Oberfächenkontamination' HYGIENE & MEDIZIN Bd. 22, Nr. 2, 1997, Seiten 77 - 92

## Beschreibung

Die vorliegende Erfindung betrifft ein Modul für mikrobiologische Beurteilungen, mit einem Aufnahmeglied für Mikroorganismen und mit einer Hülle, in welcher das Aufnahmeglied untergebracht ist, wobei dieses Modul so ausgeführt ist, dass es sich zur Beurteilung der Mikrobiologie auch von schief oder sogar vertikal verlaufenden Flächen eignet.

Ein solches Modul ist aus der Veröffentlichung "Eine neue Methode zur Untersuchung der mikrobiellen Oberflächenkontamination" in der Fachzeitschrift: Hygiene & Medizin, Ed. 22, 1997, num. 2, Seiten 77-92 bekannt. Die Hülle dieses Modules besteht aus zwei quadratischen und praktisch gleich grossen Platten aus Medizinalpapier, deren Ränder miteinander verbunden sind. Im Inneren einer solchen Hülle liegt das Glied zur Aufnahme von Mikroorganismen, welches die Form einer Scheibe aus einem geeigneten Material hat. Das Aufnahmeglied ist in der Hülle lose angeordnet und in der Hülle somit im wesentlichen frei beweglich. Wenn man das Aufnahmeglied der Einwirkung eines Mikroorganismen aufweisenden Fluids aussetzen will, dann muss man die Hülle aufreissen, das Aufnahmeglied der Hülle entnehmen und an jenem Ort anbringen, dessen mikrobiologische Beurteilung durchgeführt werden soll.

Beim Berühren des Aufnahmegliedes, um dieses auf den Einsatzort zu bringen, besteht die Gefahr einer Kontamination des Aufnahmegliedes durch die verwendete Pinzette, durch die Hand der diese Untersuchung durchführenden Person oder ähnlich. Diese Kontamination kann die Resultate der mikrobiologischen Untersuchung verfälschen. Ein weiteres Problem entsteht, wenn das Aufnahmeglied an einer schiefen oder vertikal verlaufenden Fläche oder sogar an der Unterseite eines Gegenstandes angebracht werden soll. In solchen Fällen muss die untere bzw. rückwärtige Seite des Aufnahmegliedes zunächst mit einem Klebstoff versehen werden, bevor das Aufnahmeglied am Einsatzort angebracht werden kann. Bei Verwendung von Klebstoff besteht nicht nur die Gefahr der bereits erwähnten Kontamination. Denn es besteht auch die Gefahr, dass der betreffende Gegenstand, wenn es sich beispielsweise um ein altes Gemälde handelt, durch die Feuchtigkeit aus dem Klebstoff Schaden nehmen kann.

Die Aufgabe der vorliegenden Erfindung ist, die genannten sowie noch weitere Nachteile des Standes der Technik zu beseitigen.

Diese Aufgabe wird beim Modul der eingangs genannten Gattung erfindungsgemäss so gelöst, wie dies im kennzeichnenden Teil des Patentanspruchs 1 definiert ist.

Nachstehend werden Ausführungsformen der vorliegenden Erfindung anhand der beiliegenden Zeichnungen näher erläutert. Es zeigt:
Fig. 1 in einer Draufsicht eine erste Ausführungsform des vorliegenden Moduls,
Fig. 2 in einem vertikalen Schnitt A-A das Modul aus Fig. 1,
Fig. 3 in einer Draufsicht einen Ausschnitt aus einer zweiten Ausführungsform des vorliegenden Moduls und
Fig. 4 in einer Draufsicht eine dritte Ausführungsform des vorliegenden Moduls.

Das Modul umfasst eine Hülle 1, in welcher sich ein Glied 2 für die Aufnahme von Mikroorganismen befindet. Dieses Aufnahmeglied 2 hat im dargestellten Fall die Form einer Scheibe mit einer kreisförmigen Umfangskontur. Diese Umfangskontur kann jedoch auch mehr- bzw. vieleckförmig sein. Die Scheibe 2 ist aus einem für die Aufnahme von Mikroorganismen geeigneten Material, beispielsweise aus Nitrozellulose. Eine solche Aufnahmescheibe 2 wird in der Fachliteratur auch als Membrane bezeichnet.

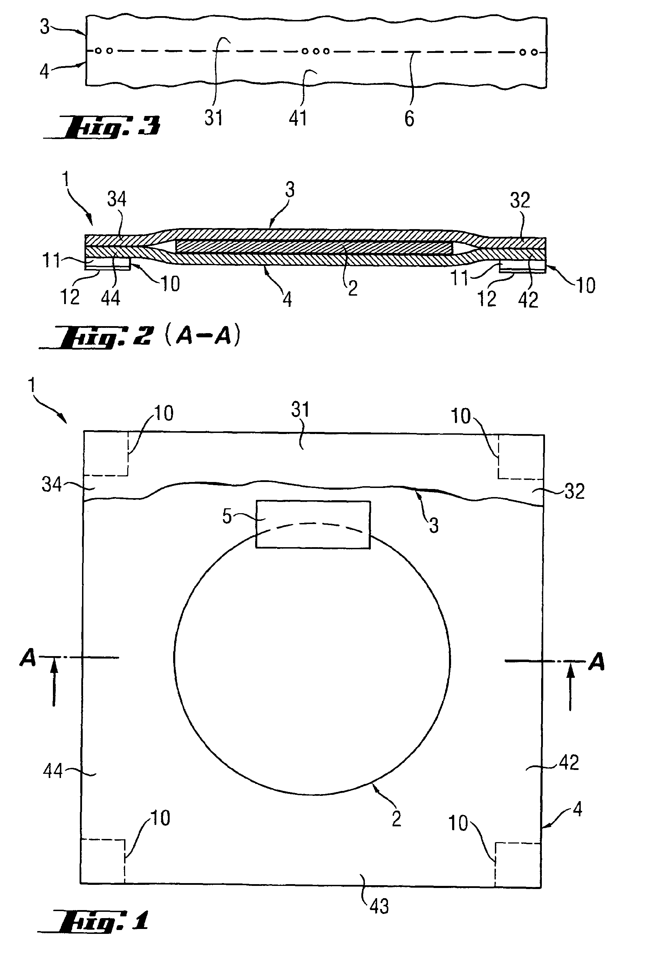

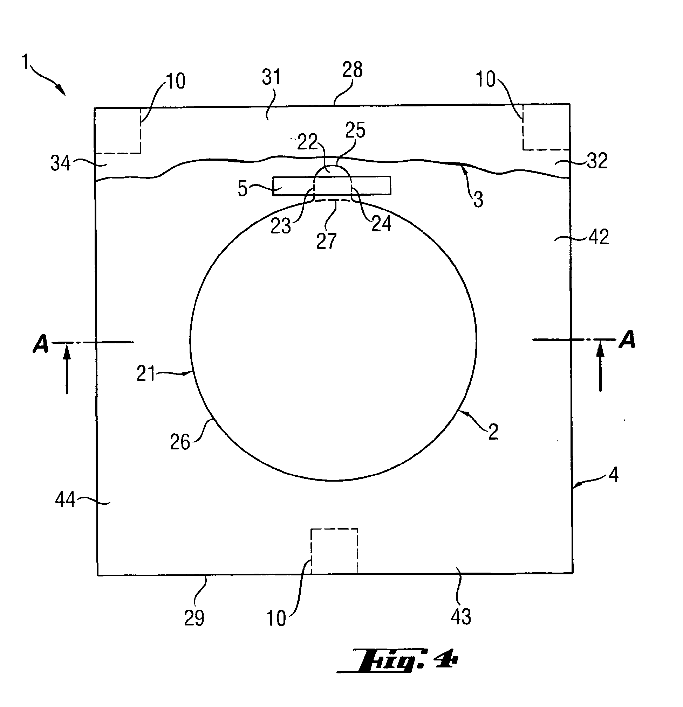

Die Hülle 1 der in Fig. 1 und 2 dargestellten Ausführungsform des vorliegenden Moduls umfasst zwei Platten 3 und 4, welche aufeinander liegen und welche beispielsweise aus dem sogenannten Medizinalpapier sind. In Fig. 1 ist ein grösserer Teil der ersten bzw. oberen Platte 3 abgebrochen, damit der Inhalt der Hülle besser ersichtlich ist. Diese obere Platte 3 kann aus einem durchsichtigen Material sein. Die Platten 3 und 4 können eine kreisförmige oder eine mehreckförmige Umfangskontur haben. Im dargestellten Fall haben die Platten 3 und 4 eine quadratische Kontur. Die obere Platte 3 weist vier Randpartien auf, von welchen nur drei Randpartien 31, 32 und 34 in Fig. 1 und 2 dargestellt sind. Die zweite bzw. untere Platte 4 weist ebenfalls vier Randpartien 41, 42, 43 und 44 auf.

Die Unterseite der Randpartien der oberen Platte 3 liegt auf der Oberseite der Randpartien der unteren Platte 4 auf. Die sich in dieser Weise gegenseitig zugeordneten Oberflächen der Randpartien der Platten 3 und 4 sind in einer an sich bekannten Weise miteinander verbunden. Zu diesem Zweck kann beispielsweise ein an sich bekannter Klebstoff verwendet werden. Die Verbindung der Randpartien kann jedoch auch mit Hilfe eines an sich bekannten Formschlusses erreicht werden. Dieser Formschluss entsteht, wenn zumindest eine tiefe Sicke (nicht dargestellt) in die aufeinander liegenden Randpartien der Platten 3 und 4 eingedrückt wird.

Die Fläche der Aufnahmescheibe bzw. der Membrane 2 ist kleiner als die Fläche der Hüllenplatten 3 und 4. Die Aufnahmescheibe 2 befindet sich zwischen den Hüllenplatten 3 und 4, und zwar etwa im mittleren Bereich dieser Platten 3 und 4, wie dies aus Fig. 2 ersichtlich ist. Wenn die Verbindung der Randpartien der Platten 3 und 4 fluiddicht ausgeführt ist, dann kann die Aufnahmescheibe 2 in einer solchen Hülle 1 steril aufbewahrt werden.

Eine der Grossflächen der Aufnahmescheibe 2 haftet auf einer der Hüllenplatten 4 stärker als auf der anderen Hüllenplatte 3. Eine solche, ungleiche bzw. asymmetrische Verbindung der Scheibe 2 mit der Hülle 1 ist so ausgeführt, dass die Aufnahmescheibe 2 auf einer der Hüllenplatte 4 haften bleibt, wenn die Hülle 1 geöffnet wird, d.h. wenn die andere Hüllenplatte 3 von der die Scheibe 2 tragenden Hüllenplatte 4 entfernt wird. Die Verbindung zwischen der Scheibe 2 und der Hüllenplatte 4 ist ferner so ausgeführt, dass die Aufnahmescheibe 2 sich dann später von der Hüllenplatte 4 entfernen lässt, ohne dass die Aufnahmescheibe 2 dabei zerstört oder beschädigt wird.

Zu diesem Zweck kann jene Grossfläche der Aufnahmescheibe 2, welche der Hüllenplatte 4 zugeodnet werden soll, so behandelt werden, dass eine beträchtliche Menge von elektrostatischen Ladungen sich zwischen dieser Grossfläche der Scheibe 2 und dem darunter liegenden Bereich der Hüllenplatte 4 sammelt und dadurch die Scheibe 2 auf der Platte 4 hält. Anstelle einer solschen Behandlung kann Klebstoff, vorteilhaft punktweise, zwischen der genannten Grossfläche der Scheibe 2 und der darunter liegenden Innenfläche der Hüllenplatte 4 angeordnet sein. Gemäss Fig. 1 ist ein Klebstreifen 5 verwendet, um eine lösbare Vebindung zwischen der Aufnahmescheibe 2 und der Hüllenplatte 4 herzustellen. Dieser Klebstreifen 5 ist so breit ausgeführt und dabei so angeordnet, dass er einerseits auf einem Abschnitt des Randes der Aufnahmescheibe 2 und andererseits auf jenem Bereich der Hüllenunterplatte 4 aufliegt und haftet, welcher zum genannten Randabschnitt der Scheibe 2 benachbart liegt.

Jene Hüllenplatte 4, auf welcher die Aufnahmescheibe 2 haftet bzw. stärker haftet, ist mit Mitteln 10 versehen, welche das Anbringen dieser Hüllenplatte 4 auf der Oberfläche eines Gegenstandes (nicht dargestellt) ermöglichen. Diese Oberfläche kann auch schräg oder sogar vertikal verlaufen oder es kann sich um eine gegen abwärts hin gerichtete Fläche handeln. Im dargestellten Fall sind die Haftmittel 10 an der Aussenseite der Hüllenplatte 4 angeordnet. Das jeweilige Haftmittel 10 ist als ein flacher und beidseitig haftender bzw. klebender Materialabschnitt 11 ausgeführt. Ueber eine seiner Haftflächen bzw. Klebseiten ist dieser Materialabschnitt 11 auf der Unterseite der die Scheibe 2 tragenden Hüllenplatte 4 aufgeklebt. Die andere Klebfläche des Materialabschnittes 11 ist bis kurz vor dem Anbringen des Moduls an einem Gegenstand mit einem Schutzstreifen 12 zugedeckt. Ein solcher Schutzstreifen 12 ist in der Regel aus Wachspapier und er wird erst vor dem Anbringen des Moduls am Gegenstand vom Klebekissen 11 entfernt.

Im dargestellten Fall sind vier solche Haftstellen bzw. Haftmittel 10 vorgesehen, wobei je eines dieser Haftmittel 10 sich im Bereich einer der Eckpartien der Hülle 1 befindet. Es versteht sich jedoch, dass solche Haftmittel 10 sich auch anderswo auf der Unterseite der tragenden Hüllenplatte 4 befinden können.

Bei der Anwendung dieses Moduls werden zunächst die Schutzstreifen 12 von den Klebpolstern 11 entfernt, sodass das Modul über die äusseren Klebflächen der Klebpolster 11 an der gewünschten Stelle eines Gegenstandes angebracht bzw. angeklebt werden kann. Dann wird die obere Hüllenplatte 3 des Moduls von der unteren Hüllenplatte 4 entfernt bzw. weggerissen, wodurch jene Grossfläche der Aufnahmescheibe 2 frei und zur Aufnahme von Mikroorganismen bereit wird, welche von der tragenden Hüllenplatte 4 abgewandt ist. Nachdem eine vorgegebene Zeitspanne für das Sammeln von Mikroorganismen ablief, wird die Aufnahmescheibe 2 von der unteren Hüllenplatte 4 entfernt und in eine Petri-Schale gelegt. Die Petri-Schale wird zusammen mit der kontaminierten Scheibe 2 in einem Brutkasten zur weiteren Behandlung untergebracht.

Zum Uebertragen in die Petri-Schale kann man die Hüllenplatte 4 erfassen und diese zusammen mit der darauf haftenden Aufnahmescheibe 2 in die Petri-Schale legen. Dies bietet den wichtigen Vorteil, dass man die mit den Mikroorganismen bereits kontaminierte Aufnahmescheibe 2 gar nicht berühren muss. Es ist jedoch auch möglich, die Aufnahmescheibe 2 zunächst von der tragenden Hüllenplatte 4 wegzunehmen, beispielsweise mit Hilfe einer Pinzette, und die Scheibe 2 dann allein in die Petri-Schale zum Bebrüten zu legen.

Fig. 3 zeigt in Draufsicht einen Ausschnitt aus einer zweiten Ausführung des vorliegenden Moduls. Bei dieser zweiten Ausführung des Moduls bilden die Hüllenplatten 3 und 4 ein Stück bzw. einen Streifen. Im Bereich des Ueberganges zwischen diesen Hüllenplatten 3 und 4 bzw. zwischen den Randpartien 31 und 41 dieser Hüllenplatten 3 und 4 ist eine Perforation 6 ausgeführt, welche praktisch senkrecht zur Länge des aus den beiden Hüllenplatten 3 und 4 bestehenden Papierstreifens verläuft. Dabei befinden sich diese Perforation 6 praktisch in der Mitte der Länge dieses Papierstreifens.

Wenn die Scheibe 2, wie in Fig. 1 dargestellt, mit Hilfe des Klebstreifens 5 auf der Tragplatte 4 befestigt ist, dann kann es vorkommen, dass ein Teil des durch den Klebstreifen 5 überdeckten Randes der Scheibe 2 am Klebstreifen 5 haften bleibt und vom übrigen Teil der Scheibe 2 weggerissen wird. Die Grösse der Fläche des abgerissenen Randabschnittes der Scheibe 2 hängt von verschiedenen Umständen ab und folglich ist es unmöglich, im voraus zu bestimmen, wie gross die Fläche des weggerissenen Randabschnittes sein wird. Wenn unterschiedlich grosse Abschnitte des Scheibenrandes am Klebstreifen hängen bleiben und somit vom übrigen Teil der Scheibe abgetrennt werden, dann weisen die weiter zu behandelnden Scheiben unterschiedlich grosse Flächen auf. Da die Grösse der gemessenen Kontamination auch von der Grösse der Scheibenfläche abhängt, dürfte es einleuchten, dass die gemessenen Kontaminationswerte durch die variierende Grösse der Fläche der Scheiben 2 beeinflusst werden.

Dieser Nachteil wird durch die Ausführung des Moduls gemäss Fig. 4 beseitigt. Der Grundkörper 21 der Scheibe 2 weist einen Ausläufer 22 aus dem Material der Scheibe 2 auf. Dieser Ausläufer oder Lappen 22 hat im dargestellten Fall die Form einer Zunge, mit zwei praktisch parallel zueinander verlaufenden Seitenkanten 23 und 24, welche sich einerends an die Umfangskante 26 der Scheibe 2 anschliessen. Die anderen Enden der Seitenkanten 23 und 24 sind mit Hilfe einer Querkante 25 miteinander verbunden, welche im dargestellten Fall bogenförmig ist und welche jedoch auch geradlinig verlaufen kann. Die Längsrichtung des Klebstreifens 5 verläuft praktisch senkrecht zur Längsrichtung des Materialausläufers 22 und im dargestellten Fall somit senkrecht zu den Seitenkanten 23 und 24 des Ausläufers 22. Der Befestigungsstreifen 5 liegt nur auf dem Ausläufer 22 auf.

Unter Umständen kann zwischen jenen Stellen des Scheibenkörpers 21, wo sich die Seitenkanten 23 und 24 an die Umfangskante 26 der Scheibe 2 anschliessen, eine Sollbruchstelle 27 ausgeführt sein. Diese Sollbruchstelle 27 kann als eine Perforation im Material des Scheibengrundkörpers 21 ausgeführt sein. Die Sollbruchstelle 27 stellt sicher, dass der Ausläufer 22 immer an derselben Stelle des Scheibengrundkörpers 21 von diesem abgetrennt wird. Folglich weisen die Scheiben 2, welche nach der Entfernung der Ausläufer 22 weiter behandelt werden, dieselbe Fläche auf.

Gemäss Fig. 4 weist die Hülle 1 nur drei Befestigungskissen 10 auf. Zwei dieser Kissen 10 sind einer der Seitenkanten 28 der Hülle 1 zugeordnet und jeder von diesen befindet sich, wie bisher, in einer der Eckpartien der Hülle 1. Der dritte Befestigungskissen 10 ist der gegenüberliegenden Seitenkante 29 der Hülle 1 zugeordnet und dieses Kissen 10 befindet sich praktisch in der Mitte der Länge dieser Seitenkante 29.

Ein solches Modul ist insbesondere zur Beurteilung der Mikrobiologie geeignet, wozu das Innere des Moduls steril ist. Um diesen sterilen Zustand während langer Zeit aufrechtzuerhalten, sind die Ränder der Platten 3 und 4 rundum verschweisst. Das Modul kann an Flächen angebracht werden, welche schief oder sogar vertikal verlaufen. Die zu beurteilenden Flächen können jedoch auch gegen abwärts hin gerichtete Flächen sein.

## Patentansprüche

1. Modul für mikrobiologische Beurteilungen, mit einem Aufnahmeglied für Mikroorganismen und mit einer Hülle, in welcher das Aufnahmeglied untergebracht ist, **dadurch gekennzeichnet, dass** dieses Modul so ausgeführt ist, dass es sich zur Beurteilung der Mikrobiologie auch von schief oder sogar vertikal verlaufenden Flächen eignet, wobei die Hülle (1) mit Mitteln (10) versehen ist, welche das Anbringen der Hülle (1) auf der Oberfläche eines Gegenstands ermöglichen, dass diese Haftmittel (10) flache und beidseitig haftende bzw. klebende Materialabschnitte bzw. Kissen (11) umfassen, dass das jeweilige Kissen (11) über eine seiner Haftflächen bzw. Klebseiten auf der Hülle (1) aufgeklebt ist und dass die andere Klebfläche des Kissens (11) mit einem Schutzstreifen (12) zugedeckt ist.

2. Modul nach Patentanspruch 1, **dadurch gekennzeichnet, dass** die Haftmittel (10) zweckmässigerweise im Randbereich der Hülle (1) angeordnet sind,

3. Modul nach Patentanspruch 1, **dadurch gekennzeichnet, dass** die Hülle (1) zwei Platten (3,4) umfasst, welche aufeinander liegen und welche beispielsweise aus dem sogenannten Medizinalpapier sein können, dass die Hüllenplatten (3,4) eine kreisförmige oder eine mehreckförmige Umfangskontur haben können, und dass die aufeinander liegenden Randpartien der Platten (3,4) lösbar miteinander verbunden sind.

4. Modul nach Patentanspruch 3, **dadurch gekennzeichnet, dass** das Aufnahmeglied (2) als eine Scheibe aus einem für die Aufnahme von Mikroorganismen geeigneten Material, beispielsweise aus Nitrozellulose, ausgeführt ist, dass diese Scheibe eine kreisförmige oder vieleckförmige Umfangskontur haben kann, dass die Fläche der Scheibe (2) kleiner ist als die Fläche der Hüllenplatten (3,4) und dass die Aufnahmescheibe (2) sich zwischen den Platten (3,4) befindet, und zwar etwa im mittleren Bereich dieser Hüllenplatten (3,4).

5. Modul nach Patentanspruch 4, **dadurch gekennzeichnet, dass** das Aufnahmeglied (2) an bzw. auf einer der Platten (4) der Hülle (1) des Moduls entfernbar angebracht ist und dass diese Hüllenplatte (4) mit den Mitteln (10) aufweist, mit deren Hilfe sich das Aufnahmeglied (2) an einem Gegenstand lösbar anbringen lässt.

6. Modul nach Patentanspruch 4, **dadurch gekennzeichnet, dass** eine der Grossflächen der Aufnahmescheibe (2) auf einer der Hüllenplatten (4) stärker haftet als auf der anderen Hüllenplatte (3) und dass die Haftkraft dieser Verbindung so eingestellt ist, dass die Aufnahmescheibe (2) auf der diese tragenden Hüllenplatte (4) haften bleibt, wenn die andere Hüllenplatte (3) entfernt wird, und dass die Aufnahmescheibe (2) sich von der tragenden Hüllenplatte (4) dann entfernen lässt.

7. Modul nach Patentanspruch 6, **dadurch gekennzeichnet, dass** eine der Grossflächen der Aufnahmescheibe (2) mit Hilfe von elektrostatischen Ladungen oder von Klebstoff auf der tragenden Hüllenplatte (4) lösbar befestigt ist, oder dass die Aufnahmescheibe (2) mit Hilfe eines Klebstreifens (5) auf der tragenden Hüllenplatte (4) befestigt ist.

8. Modul nach Patentanspruch 7, **dadurch gekennzeichnet, dass** die Haftmittel (10) an jener Grossfläche der tragenden Hüllenplatte (4) angebracht sind, welche von jener Grossfläche dieser Hüllenplatte (4) abgewandt ist, auf der die Aufnahmescheibe (2) haftet.

9. Modul nach Patentanspruch 1, **dadurch gekennzeichnet, dass** die Hüllenplatten (3,4) sich über je eine ihrer Randpartien (31,41) so zugeordnet sind, dass sie ein Stück bzw. einen Streifen bilden und dass im Bereich des Überganges zwischen diesen Hüllenplatten (3,4) eine Perforation (6) in diesem Materialstreifen ausgeführt ist, welche praktisch senkrecht zur Länge des aus den beiden Hüllenplatten (3,4) bestehenden Materialstreifens verläuft.

10. Modul nach Patentanspruch 8, **dadurch gekennzeichnet, dass** ein Ausläufer (22) von der Randpartie (26) des Grundkörpers (21) der Aufnahmescheibe (2) absteht und dass der Befestigungsstreifen (5) auf diesem Ausläufer (22) aufliegt.

## Claims

1. Microbiological diagnostical device, having a receipting member for microorganisms and having a wrapper, within which the receipting member is accommodated, **characterized in that** the present device is carried out in such a way that it is suitable for the evaluation of the microbiology of surfaces which are arranged inclined or even vertically, whereby the wrapper (1) is provided with means (10), which make it possible to mount the wrapper (1) onto the surface of an object, that said adhesive means (10) comprise material sections and/or cushions (11) which are flat and whereby the both sides thereof are adhering and/or sticking, that the respective cushion (11) is affixed over one of its adhering surfaces and/or sticking-sides on the wrapper (1), and that the other sticking surface of the cushion (11) is covered with a protecting stripe (12).

2. Device as claimed in patent claim 1, **characterized in that** the adhesive means (10) are expediently placed (1) at the border area of the wrapper (1).

3. Device as claimed in patent claim 1, **characterized in that** the wrapper (1) comprises two plates (3,4), which lie one on the another and which can be made for example of the so-called medical paper, that the outline of the wrapper plates (3,4) can be circular or a polygonal-shaped, and that the border areas of said plates which lie one on the another are detachably connected together.

4. Device as claimed in patent claim 3, **characterized in that** the receipting member (2) is made as a disc of a material which is suitable for the reception of microorganisms, for example of nitrocellulose, that this disc can have a circular or a polygon-shaped contour of its outline, that the area of the disc (2) is smaller than the area of the wrapper plates (3,4) and that the receipting disc (2) is placed between the plates (3,4), that is in the middle area of these wrapper plates (3,4).

5. Device as claimed in patent claim 4, **characterized in that** the receipting member (2) is removable placed at and/or on one of the plates (4) of the wrapper (1) of the present device and that this wrapper plate (4) is provided with said means (10) for the detachable securing the receipting member (2) at an object.

6. Device as claimed in patent claim 4, **characterized in that** one of the major surfaces of the receipting disc (2) adheres to one of the wrapper plates (4) more strongly than to the other wrapper plate (3) and that the strength of this connection is so adjusted, that the receipting disc (2) remains on wrapper plate (4) if the other wrapper plate (3) is removed, and that the receipting disc (2) can then be removed the carrying wrapper plate (4).

7. Device as claimed in patent claim 6, **characterized in that** one of the major faces of the receipting disc (2) is detachably fixed on wrapper plate (4) by electrostatic loads or by an adhesive, or that the receipting disc (2) is fixed on the wrapper plate by an adhesive tape (5).

8. Device as claimed in patent claim 7, **characterized in that** the adhesive means (10) is placed at that major face of the carrying wrapper plate (4), which is averted from that major surface of this wrapper plate (4) to which the receipting disc (2) adheres.

9. Device as claimed in patent claim 1, **characterized in that** the wrapper plates (3,4) are so attached one to the another over one of their border areas (31,41) that they form one piece and/or a stripe, and that a perforation is carried out in the material strip in the area of the transition between these wrapper plates (3,4) which extends nearly perpendicularly to the length of the material stripe consisting of the two wrapper plates (3,4).

10. Device as claimed in patent claim 8, **characterized in that** an extension (22) sticks out from the border area (26) of the basic corpus (21) of the receipting disc (2), and that the fixing stripe (5) is lying on this extension (22).

## Revendications

1. Module de diagnostic microbiologique, comprenant un organe récepteur pour des microorganismes et une coque dans laquelle l'organe récepteur est logé, **caractérisé en ce que** ce module est réalisé de telle sorte qu'il soit approprié pour le diagnostic de microbiologie sur des surfaces s'étendant aussi en biais ou même verticalement, la coque (1) étant pourvue de moyens (10) permettant de monter la coque (1) sur la surface d'un objet, **en ce que** ces moyens d'adhésion (10) comprennent des portions de matériau ou des coussins (11) plats et adhérant ou collant des deux côtés, **en ce que** chaque coussin (11) est collé sur la coque (1) par l'une de ses surfaces adhésives ou l'un de ses côtés collants, et **en ce que** l'autre surface collante du coussin (11) est recouverte d'un ruban de protection (12).

2. Module selon la revendication 1, **caractérisé en ce que** les moyens d'adhésion (10) sont disposés de manière appropriée dans la région des bords de la coque (1).

3. Module selon la revendication 1, **caractérisé en ce que** la coque (1) comprend deux plaques (3, 4) qui sont disposées l'une sur l'autre et qui peuvent par exemple provenir de ce qu'on appelle du papier médical, **en ce que** les plaques (3, 4) de la coque peuvent avoir un contour périphérique circulaire ou polygonal, et **en ce que** les parties des bords des plaques (3, 4) superposées sont connectées les unes aux autres de manière détachable.

4. Module selon la revendication 3, **caractérisé en ce que** l'organe récepteur (2) est réalisé sous la forme d'un disque en un matériau approprié pour recevoir des microorganismes, par exemple en nitrocellulose, **en ce que** ce disque peut avoir un contour périphérique circulaire ou polygonal, **en ce que** la surface du disque (2) est plus petite que la surface des plaques (3, 4) de la coque et **en ce que** le disque récepteur (2) se trouve entre les plaques (3, 4) et ce approximativement dans la région centrale de ces plaques (3, 4) de la coque.

5. Module selon la revendication 4, **caractérisé en ce que** l'organe récepteur (2) est monté de manière amovible contre ou sur l'une des plaques (4) de la coque (1) du module, et **en ce que** cette plaque (4) de la coque est pourvue des moyens (10) à l'aide desquels l'organe récepteur (2) peut être monté de manière détachable sur un objet.

6. Module selon la revendication 4, **caractérisé en ce que** l'une des grandes surfaces du disque récepteur (2) adhère plus fortement sur l'une des plaques (4) de la coque que sur l'autre plaque (3) de la coque, **en ce que** la force d'adhésion de cette connexion est ajustée de telle sorte que le disque récepteur (2) reste en adhérence sur la plaque (4) de la coque le portant, lorsque l'autre plaque (3) de la coque est enlevée, et **en ce que** le disque récepteur (2) peut alors être enlevé de la plaque (4) portante de la coque.

7. Module selon la revendication 6, **caractérisé en ce que** l'une des grandes surfaces du disque récepteur (2) est fixée de manière détachable à l'aide de charges électrostatiques ou d'adhésif sur la plaque (4) portante de la coque, ou **en ce que** le disque récepteur (2) est fixé au moyen d'un ruban adhésif (5) sur la plaque (4) portante de la coque.

8. Module selon la revendication 7, **caractérisé en ce que** les moyens d'adhésion (10) sont montés sur la grande surface de la plaque (4) portante de la coque qui est opposée à la grande surface de cette plaque (4) de la coque qui adhère au disque récepteur (2).

9. Module selon la revendication 1, **caractérisé en ce que** les plaques (3, 4) de la coque sont associées l'une à l'autre par le biais d'une de leurs parties de bords (31, 41), de telle sorte qu'elles forment un morceau ou un ruban et **en ce que** dans la région du passage entre ces plaques (3, 4) de la coque, on réalise une perforation (6) dans ce ruban de matériau qui s'étend pratiquement perpendiculairement à la longueur du ruban de matériau constitué des deux plaques (3, 4) de la coque.

10. Module selon la revendication 8, **caractérisé en ce qu'**une projection (22) fait saillie de la partie de bord (26) du corps de base (21) du disque récepteur (2) et **en ce que** le ruban de fixation (5) repose sur cette projection (22).
